(19) Europäisches Patentamt
European Patent Office
Office européen des brevets

(11) **EP 2 358 724 B1**

(12) **EUROPEAN PATENT SPECIFICATION**

(45) Date of publication and mention
of the grant of the patent:
**29.08.2012 Bulletin 2012/35**

(51) Int Cl.:
*C07D 491/08* (2006.01)   *A61K 31/46* (2006.01)
*A61P 11/00* (2006.01)

(21) Application number: **09749173.2**

(22) Date of filing: **29.10.2009**

(86) International application number:
**PCT/GB2009/002575**

(87) International publication number:
**WO 2010/052450 (14.05.2010 Gazette 2010/19)**

(54) **TIOTROPIUM BROMIDE HAVING A LOW DEGREE OF CRYSTALLINITY**

TIOTROPIUMBROMID MIT NIEDRIGEM KRISTALLINITÄTSGRAD

BROMURE DE TIOTROPIUM AYANT UN FAIBLE DEGRÉ DE CRISTALLINITÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.11.2008 IN MU23512008**

(43) Date of publication of application:
**24.08.2011 Bulletin 2011/34**

(73) Proprietor: **CIPLA LIMITED**
**Mumbai 400 008 (IN)**

(72) Inventors:
• **KANKAN, Rajendra, Narayanrao**
**Maharashtra (IN)**
• **RAO, Dharmaraj, Ramachandra**
**Maharashtra (IN)**
• **GHAGARE, Maruti**
**Maharashtra (IN)**

(74) Representative: **Cottrill, Emily Elizabeth Helen**
**A.A. Thornton & Co.**
**235 High Holborn**
**London WC1V 7LE (GB)**

(56) References cited:
WO-A-2005/042527   WO-A-2006/117299
WO-A-2007/075858   US-A- 4 684 698

## Description

### Field of the Invention

The present invention relates to tiotropium bromide having a low degree of crystallinity, a process for preparing it and pharmaceutical compositions of it.

### Background and Prior Art

Tiotropium bromide was first disclosed in EP0418716. Tiotropium bromide is a long-acting, anticholinergic bronchodilator used in the management of chronic obstructive pulmonary disease. On topical application it acts mainly on $M_3$ muscarinic receptors located in the airways to produce smooth muscle relaxation, thus producing a bronchodilatory effect. Tiotropium bromide capsules for inhalation are co-marketed by Boehringer-Ingelheim and Pfizer.

EP1326862 and EP1401445 describe monohydrate and anhydrous forms of tiotropium bromide, respectively.

WO2007/075858 discloses the amorphous form of tiotropium bromide.

WO2008/152398 describes a formulation comprising tiotropium bromide coated with polyvinylpyrrolidone (PVP). The coating requires multiple processing and may cause difficulties in scale-up due to overspray and lack of uniformity.

Pulmonary disorders are best treated when tiotropium bromide is administered in the form of inhalation. Suitable inhalation devices include metered dose inhalers ("MDIs"), dry powder inhalers and nebulizers. The manufacture of the abovementioned preparations depend upon various parameters related to the nature of the active substance. The actives can be either in crystalline or amorphous form.

The crystalline form of a drug compound may have advantages over an amorphous form. For example, a crystalline form may be more stable than an amorphous form, both before and during formulation and during subsequent storage. The bioavailability of a drug substance is affected by the physical properties of a drug substance such as by crystallinity, particle size, hygroscopicity, bulk density, flow characteristic etc. Crystallization is a convenient method for purification of a drug substance on a larger scale, than other known techniques of purification such as chromatography. Also, when formulating a crystalline drug substance for delivery by inhalation, it is generally easier to mill or micronise a crystalline form to a respirable size (generally considered as particles less than 5 microns in diameter).

Crystalline forms are generally considered to be more stable but to tend to dissolve with slight difficulty; compared to the amorphous form which may have higher solubility but is generally less stable as it can convert to the crystalline form. Due to the absence of an ordered crystal lattice, the amorphous form requires minimal energy and thus provides the maximal solubility advantage as compared to the crystalline and hydrated forms of a drug. The apparent solubility and dissolution advantage offered by these systems is a vital approach to enhance the bioavailability of drugs. However, the limitations of amorphous systems such as physical instability and higher chemical reactivity, act as a hurdle in their extensive commercialization.

Therefore, it is important to understand the molecular and thermodynamic properties that contribute to the solubility and stability of drugs and hence there is a need to develop a drug substance with physical properties that are suitable for pharmaceutical use.

### Objects of the Invention

An object of the present invention is to provide tiotropium bromide having a low degree of crystallinity.

Another object of the present invention is to provide a tiotropium bromide-polyvinylpyrrolidone (PVP) complex.

Yet another object of the present invention is to provide a simple process for the preparation of tiotropium bromide having a low degree of crystallinity.

Still another object of the present invention is to provide tiotropium bromide with a high degree of stability and dose uniformity.

A further object of the present invention is to prepare a pharmaceutical composition containing tiotropium bromide having a low degree of crystallinity.

### Summary of the Invention

According to a first aspect of the present invention, there is provided a complex of tiotropium bromide and polyvinylpyrrolidone (PVP). The PVP may be selected from the group consisting of PVP-K-12, PVP-K-15, PVP-K-17, PVP-K-25, PVP-K-30, PVP-K-60 and PVP-K-90. Suitably, the PVP may be PVP-K-25. The PVP may have a molecular weight ranging from 2500 to 1,200,000.

Advantageously, the tiotropium bromide has a degree of crystallinity lower than or equal to 75%, preferably lower than 70%.

Also described is tiotropium bromide having a degree of crystallinity lower than or equal to 75%, preferably lower than or equal to 70%. The degree of crystallinity may be below 75% and greater than 0%. Preferably, the degree of crystallinity is below 70% and greater than 0%. Suitably, the degree of crystallinity ranges from 10% to 75%, more preferably from 10% to 70%, still more preferably from 30% to 60%. The percent degree of crystallinity is determined as described below in the detailed description of the invention.

According to another aspect of the present invention, there is provided a process for preparing a tiotropium bromide-PVP complex, the process comprising preparing a solution of tiotropium bromide, PVP and a solvent, and isolating the tiotropium bromide-PVP complex from the solution. In an embodiment, the isolation comprises concentrating the solution under vacuum to obtain a residue, and drying the residue to obtain the tiotropium bromide-PVP complex.

In an embodiment, the solvent is selected from the group consisting of acetonitrile, methanol, water, dimethyl formamide, acetone, tetrahydrofuran, dimethyl sulfoxide and mixtures thereof, preferably acetone.

In an embodiment, the amount of PVP ranges from about 0.2% to about 90% by weight of the tiotropium bromide, preferably from about 10% to about 85% by weight of the tiotropium bromide, more preferably from about 20% to about 80% by weight of the tiotropium bromide, still more preferably from about 30% to about 70% by weight of the tiotropium bromide and yet more preferably from about 40% to about 60% by weight of the tiotropium bromide. Typically, the amount of PVP in the complex is about 50% by weight of the tiotropium bromide.

In an embodiment, the tiotropium bromide is mixed with the solvent to form a reaction mixture. The reaction mixture may be heated to a suitable temperature to obtain a clear solution. PVP may then be added to the reaction mixture. The solution may be concentrated under vacuum to obtain a residue. The residue may be washed with the same solvent as was used to form the solution and dried at a suitable temperature, preferably at a temperature ranging from about 30°C to about 60°C, more preferably around 50°C, to obtain the tiotropium bromide-PVP complex.

According to another aspect of the present invention, there is provided a process for preparing a tiotropium bromide-PVP complex, the process comprising preparing a solution of tiotropium bromide, PVP and a solvent, and flash-evaporating the solvent. The solvent may be selected from the group consisting of a C1-C4 alcohol (for example, methanol, ethanol, i-propanol or butanol), acetone, water, acetonitrile, dichloromethane or mixtures thereof.

In an embodiment, the solvent is flash-evaporated by applying heat and vacuum.

In another embodiment, the flash-evaporation is carried out by spray-drying.

According to another aspect of the present invention, there is provided a process for preparing a tiotropium bromide-PVP complex, the process comprising preparing a solution of tiotropium bromide, PVP and a solvent, freeze-drying the solution and lyophilizing the freeze-dried solution. The solvent may be selected from the group consisting of water, a C1-C4 alcohol (for example, methanol, ethanol, i-propanol or butanol), acetone and tetrahydrofuran (THF).

The tiotropium bromide used in the processes of the present invention may be in any crystalline form. The tiotropium bromide used in the process of the present invention may be a hydrate form, anhydrous, a derivative thereof, or in prodrug form.

In an embodiment, the tiotropium bromide-PVP complex described above has been prepared by any one of the processes described above.

According to another aspect of the present invention, there is provided a pharmaceutical composition comprising a tiotropium bromide-PVP complex as described above together with one or more pharmaceutically acceptable excipients.

In an embodiment, the formulation is suitable for administration by inhalation. Suitably, the formulation is an aerosol.

In an embodiment, the formulation comprises the tiotropium bromide-PVP complex, at least one hydrofluoroalkane propellant and optionally one or more pharmaceutically acceptable excipients. Suitably, the or each pharmaceutically acceptable excipient is a bulking agent and/or a co-solvent. The bulking agent may be lactose. The co-solvent may be polyethylene glycol (PEG). In an embodiment, the formulation consists of the tiotropium bromide-PVP complex, lactose, and a hydrofluoroalkane such as HFA 227 (1,1,1,2,3,3,3-heptafluoropropane).

## Brief Description of the Drawings

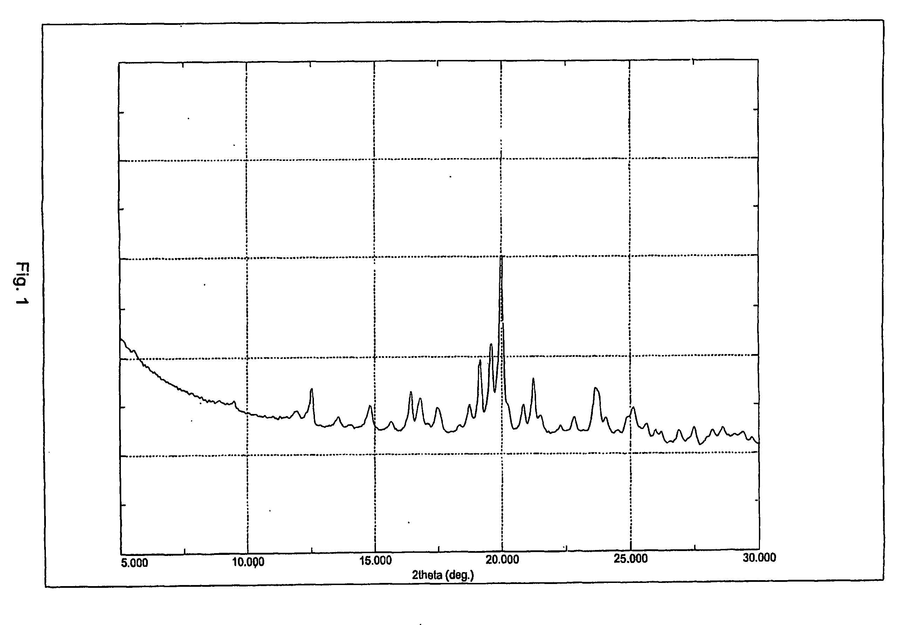

Fig. 1 shows an XRPD of a tiotropium bromide-PVP complex of the present invention

## Detailed Description of the Invention

Drugs like tiotropium that show therapeutic efficacy at very low concentrations need to be dispensed in a manner such that they provide a high degree of homogeneity and only a slight fluctuation in the dispersion characteristics.

Tiotropium bromide administered preferably by inhalation needs to fulfill certain parameters such as homogeneity of the powder mixture, reproducible release of the active in constant amounts, and low variability and stability under various environmental conditions, during production and in the final composition. These are essential requirements in order to prevent the use of compositions that contain breakdown products of the active.

The nature of tiotropium bromide in the formulation in turn has an effect on the parameters. The crystalline form is known to dissolve with difficulty while the amorphous form which, although it has a higher solubility, tends to get readily converted to the crystalline form.

Accordingly the present invention provides a form of tiotropium bromide with a high degree of solubility and enhanced absolute bioavailability while also preserving the physical and chemical stability required for formulation.

The inventors of the present invention have also surprisingly found that, when used as a carrier for tiotropium bromide, polyvinylpyrrolidone (PVP) increases the stability and solubility of tiotropium bromide by decreasing the degree of crystallinity of the tiotropium bromide.

The tiotropium bromide-PVP complex of the present invention exists not as a mere physical mixture, but as a complex with its own unique physical/chemical properties.

PVP is a homopolymer of N-vinyl-2-pyrrolidone on which the tiotropium bromide gets adsorbed. Complexation of tiotropium bromide with PVP is a non-covalent stacking type of association between non-polar areas of tiotropium bromide and PVP. The complex shows no agglomeration of the drug particles as compared to the crystalline form and has better stability than the amorphous form. The complex shows good aerosol dispersion and higher solubility for inhalation drug delivery.

The tiotropium bromide complex of the present invention has unique physical and chemical properties, which properties are significantly different from those of a physical mixture of tiotropium bromide and PVP. A physical mixture of tiotropium bromide tends to form an agglomeration of the drug substance and PVP. The physical mixture does not have any synergistic effect whereas the complex of the present invention has a significant effect on the properties and stability of the drug substance thereby affecting its bioavailability and efficacy which effect is not seen in a physical mixture.

The present invention thus provides tiotropium bromide having a low degree of crystallinity which is more suitable for pharmaceutical preparations as compared to pure crystalline and amorphous forms.

In another aspect, the present invention provides tiotropium bromide having a low degree of crystallinity that may be obtained by complexing with polyvinylpyrrolidone (PVP).

Polyvinylpyrrolidones (PVP) are cross-linked polymers classified either on the basis of K-value as PVP-K-12, PVP-K-15, PVP-K-17, PVP-K-25, PVP-K-30, PVP-K-60, PVP-K-90 or molecular weight as ranging from PVP-2500 to 1,200,000.

In an embodiment, the present invention provides a method for preparing tiotropium bromide having a low degree of crystallinity. The method comprises mixing tiotropium bromide with a suitable solvent. The reaction mixture may be heated to a suitable temperature to obtain a clear-solution. PVP is then added to the reaction mixture. This solution is concentrated under vacuum to obtain a residue. The residue is washed with the same solvent and dried at a suitable temperature preferably 50°C to obtain a tiotropium bromide-PVP complex.

The solvent used In the above process may be selected from acetonitrile, methanol, water, dimethyl formamide, acetone, tetrahydrofuran or dimethyl sulfoxide, preferably acetone.

In an embodiment, the amount of PVP should be between about 0.2% and about 90% by weight of the tiotropium bromide, preferably between about 10% and about 80% by weight of the tiotropium bromide, more preferably, between about 20% and about 70% by weight of the tiotropium bromide, still more preferably between about 30% and about 60% by weight of the tiotropium bromide, most preferably around 50% by weight of the tiotropium bromide.

Alternatively, tiotropium bromide having a low degree of crystallinity can be isolated by "flash-evaporating" the solvent. A flash-evaporating technique with respect to this invention means removal of the solvent by applying heat and vacuum. Preferably, the temperature in the heating step ranges from about 40°C to about 70°C, preferably from about 45°C to about 60°C, most preferably from about 50°C to about 55°C.

In an embodiment, flash evaporation is carried out by spray-drying.

Alternatively, isolation of tiotropium bromide having a low degree of crystallinity can be carried out by lyophilization.

The tiotropium bromide used in the process of the present invention may be in any crystalline form. The tiotropium bromide used in the process of the present invention may be a hydrate form, anhydrous, a derivative thereof, or in prodrug form.

In another aspect, the present invention provides a pharmaceutical composition comprising a tiotropium bromide-PVP complex and one or more pharmaceutically acceptable carriers.

In an embodiment, the degree of crystallinity of the tiotropium bromide-PVP complex can be measured with X-ray powder diffraction (XRPD). For this analysis, a thin layer of the triturated sample is smeared onto cut silicon single crystal zero background holder. Cu KV radiation and constant or automatic anti scatter and divergence slits are used to obtain a diffractogram with 20 values from $3^0$ to at least $40^0$.

The degree of crystallinity is calculated with the formula:

$$\% \text{ Crystallinity} = 100\, A\,/\,(A+B-C)$$

A = total area of peak arising from diffraction from the crystalline fraction of the sample (i.e. crystalline region)

B = total area below area A (i.e. amorphous and background region)

C = background area (due to air scattering, fluorescence, equipment, etc)

Area calculations are performed for a $2\theta$ range of $3^0$-$4.0^0$.

Background area (arising only from the instrument parameters) is subtracted using manual spline, area of this profile from $3^0$-$40\ ^0 2\theta$ is calculated which gives the area for amorphous and crystalline areas of the sample i.e. (A+B-C).

The XRD pattern is subjected to normal background subtraction that eliminates background area arising due to both amorphous and instrumental parameter. This area is taken as the crystalline area of the pattern (A).

In an embodiment, there is provided tiotropium bromide having a degree of crystallinity which is below 75%. More preferably, the degree of crystallinity is below 70%. The degree of crystallinity may be below 75% and greater than 0%. Preferably, the degree of crystallinity is below 70% and greater than 0%. Suitably, the degree of crystallinity ranges from 10% to 75%, more preferably from 10% to 70%, still more preferably from 30% to 60%. The percent degree of crystallinity is determined as described above.

There follow, by way of non-restrictive explanation of the present invention, the following examples.

**Example 1:**

TTB-PVP Complex

5 g of tiotropium bromide was introduced into a reaction vessel. Acetone was added. The reaction mixture was heated to a temperature of 50-55°C. Water (15 ml) was added to the reaction mixture to obtain a clear solution. To this solution, 2.5 g of PVP-K-25 was added. The solution was concentrated under vacuum to obtain a residue. The residue was washed with acetone (15 ml) and dried under vacuum at 50°C to obtain the title complex (6 g)

The XRPD of the Example 1 complex is shown in Figure 1. From this XRPD, and the formula quoted above, the degree of crystallinity was calculated as being 58.35%.

**Example 2:**

TTB-PVP Complex

5 g of tiotropium bromide monohydrate was introduced into a reaction vessel. Water (15 ml) was added to the reaction mixture to obtain a clear solution. To this solution, 2.5 g of PVP-K-17 was added. The solution was concentrated under vacuum to obtain a residue. The residue was washed with acetone (15 ml) and dried under vacuum at 50°C to obtain the title complex (6 g).

Example 3:

TTB-PVP Complex

5 g of tiotropium bromide monohydrate was introduced into a reaction vessel. Acetone was added. The reaction mixture was heated to a temperature of 50-55°C. Water (15 ml) was added to the reaction mixture to obtain a clear solution. To this solution, 0.25 g of PVP-K-25 was added. The solution was concentrated under vacuum to obtain a residue. The residue was washed with acetone (15 ml) and dried under vacuum at 50°C to obtain the title complex (5.2 g) (% crystallinity - 67.42%).

**Example 4:**

TTB-PVP Complex Formed by Lyophilization

5 g of tiotropium bromide and 2.5 g of polyvinylpyrrolidone (PVP) were stirred in 50 ml of water at 25-30°C until dissolved. The solution was quick-frozen in a dry ice bath. The solution was lyophilized at a condenser temperature of approximately 0°C in the presence of a high vacuum. The resultant title complex was obtained in the form of a solid (5.8 g).

### Example 5:

TTB-PVP-K-25 Complex Formed by Evaporation

2.5 g of tiotropium bromide and 1.25 g of polyvinylpyrrolidone (PVP) were stirred in 10 ml of ethanol at 25-30°C until dissolved. The solution was poured into a polyethylene tray and the ethanol evaporated in a vacuum oven in the presence of a nitrogen stream. The resultant dry solid title product was isolated (2.5 g).

### Example 6:

TTB-PVP-K-17 Complex Formed by Spray Drying

2.5 g of tiotropium bromide was dissolved in 25 ml of methanol. 1.25 g of PVP was dissolved in methanol (15 ml). The two solutions were mixed and spray dried. The following parameters were used:

Instrument - Labultima LU-222 ADVANCE SPRAY DRYER
Temperature - 50-55°C
Vacuum - 40mmWC
$N_2$ pressure -1-2 kg
Feeding rate - 3ml/min.

The solid product was collected in a collector (2.0 g).

### Example 7:

Pharmaceutical formulation of tiotropium bromide-PVP complex

| | |
|---|---|
| Tiotropium bromide-PVP complex | 1.8 mg |
| Lactose | 1.8 mg |
| HFA[1] - 227 | Q.S. |
| [1]HFA = 1,1,1,2,3,3,3-heptafluoropropane | |

Lactose was added to a canister containing the tiotropium bromide-PVP complex. The canister was crimped with the metered valve and the propellant charged in the canister.

It will be appreciated that the invention may be modified within the scope of the appended claims.

## Claims

1.  A complex of tiotropium bromide and polyvinylpyrrolidone (PVP).

2.  A complex according to claim 1, wherein the PVP is selected from the group consisting of PVP-K-12, PVP-K-15, PVP-K-17, PVP-K-25, PVP-K-30, PVP-K-60 and PVP-K-90.

3.  A complex according to claim 1, wherein the PVP has a molecular weight ranging from 2500 to 1,200,000.

4.  A complex according to claim 1, 2 or 3, wherein the tiotropium bromide has a degree of crystallinity lower than or equal to 75%, preferably the tiotropium bromide has a degree of crystallinity lower than or equal to 70%.

5.  A process for preparing a tiotropium bromide-PVP complex, the process comprising preparing a solution of tiotropium bromide, PVP and a solvent, concentrating the solution under vacuum to obtain a residue, and drying the residue to obtain the tiotropium bromide-PVP complex.

6.  A process according to claim 5, wherein the solvent is selected from the group consisting of acetonitrile, methanol, water, dimethyl formamide, acetone, tetrahydrofuran and dimethyl sulfoxide.

7. A process according to claim 5 or 6, wherein the amount of PVP ranges from about 0.2% to about 90% by weight of the tiotropium bromide, preferably the amount of PVP ranges from about 40% to about 60% by weight of the tiotropium bromide.

8. A process for preparing a tiotropium bromide-PVP complex, the process comprising preparing a solution of tiotropium bromide, PVP and a solvent, and flash-evaporating the solvent.

9. A process according to claim 8, wherein the solvent is flash-evaporated by applying heat and vacuum.

10. A process according to claim 8, wherein the flash-evaporation is carried out by spray-drying.

11. A process for preparing a tiotropium bromide-PVP complex, the process comprising preparing a solution of tiotropium bromide, PVP and a solvent, freeze-drying the solution and lyophilizing the freeze-dried solution.

12. A pharmaceutical composition comprising a tiotropium bromide-PVP complex according to any one of claims -1 to 4, together with one or more pharmaceutically acceptable excipients.

13. A pharmaceutical composition according to claim 12, wherein the formulation is suitable for administration by inhalation.

14. A pharmaceutical composition according to claim 12 or 13, wherein the formulation is an aerosol.

15. A pharmaceutical composition according to claim 12, 13 or 14, wherein the formulation comprises a propellant.

**Patentansprüche**

1. Komplex aus Tiotropiumbromid und Polyvinylpyrrolidon (PVP).

2. Komplex nach Anspruch 1, wobei das PVP aus der Gruppe ausgewählt ist, bestehend aus PVP-K-12, PVP-K-15, PVP-K-17, PVP-K-25, PVP-K-30, PVP-K-60 und PVP-K-90.

3. Komplex nach Anspruch 1, wobei das PVP ein Molekulargewicht im Bereich von 2500 bis 1.200.000 aufweist.

4. Komplex nach Anspruch 1, 2 oder 3, wobei das Tiotropiumbromid einen Kristallinitätsgrad niedriger als oder gleich 75 % aufweist, das Tiotropiumbromid bevorzugt einen Kristallinitätsgrad niedriger als oder gleich 70 % aufweist.

5. Verfahren zum Herstellen eines Tiotropiumbromid-PVP-Komplexes, wobei das Verfahren das Herstellen einer Lösung aus Tiotropiumbromid, PVP und einem Lösungsmittel, das Konzentrieren der Lösung unter Vakuum zum Erhalt eines Rückstands und Trocknen des Rückstands zum Erhalt des Tiotropiumbromid-PVP-Komplexes umfasst.

6. Verfahren nach Anspruch 5, wobei das Lösungsmittel aus der Gruppe ausgewählt ist, bestehend aus Acetonitril, Methanol, Wasser, Dimethylformamid, Aceton, Tetrahydrofuran und Dimethylsulfoxid.

7. Verfahren nach Anspruch 5 oder 6, wobei die PVP-Menge im Bereich von ca. 0,2 bis ca. 90 Gew.% des Tiotropiumbromids liegt, die PVP-Menge bevorzugt im Bereich von 40 bis ca. 60 Gew.-% des Tiotropiumbromids liegt.

8. Verfahren zum Herstellen eines Tiotropiumbromid-PVP-Komplexes, wobei das Verfahren das Herstellen einer Lösung aus Tiotropiumbromid, PVP und einem Lösungsmittel und die Flash-Verdampfung des Lösungsmittels umfasst.

9. Verfahren nach Anspruch 8, wobei das Lösungsmittel durch Aufbringen von Wärme und Vakuum flash-verdampft wird.

10. Verfahren nach Anspruch 8, wobei die Flash-Verdampfung durch Sprühtrocknen durchgeführt wird.

11. Verfahren zur Herstellung eines Tiotropiumbromid-PVP-Komplexes, wobei das Verfahren das Herstellen einer Lösung aus Tiotropiumbromid, PVP und einem Lösungsmittel, das Gefriertrocknen der Lösung und Lyophilisieren der gefriergetrockneten Lösung umfasst.

**12.** Pharmazeutische Zusammensetzung, umfassend einen Tiotropiumbromid-PVP-Komplex nach einem der Ansprüche 1 bis 4, zusammen mit einem oder mehr pharmazeutisch verträglichen Hilfsstoff(en).

**13.** Pharmazeutische Zusammensetzung nach Anspruch 12, wobei die Formulierung zur Verabreichung durch Inhalation geeignet ist.

**14.** Pharmazeutische Zusammensetzung nach Anspruch 12 oder 13, wobei die Formulierung ein Aerosol ist.

**15.** Pharmazeutische Zusammensetzung nach Anspruch 12, 13 oder 14, wobei die Formulierung ein Treibmittel umfasst.


**Revendications**

**1.** Complexe de bromure de tiotropium et de polyvinylpyrrolidone (PVP).

**2.** Complexe selon la revendication 1, dans lequel la PVP est choisie dans le groupe constitué par la PVP-K-12, la PVP-K-15, la PVP-K-17, la PVP-K-25, la PVP-K-30, la PVP-K-60 et la PVP-K-90.

**3.** Complexe selon la revendication 1, dans lequel la PVP a une masse moléculaire allant de 2 500 à 1 200 000.

**4.** Complexe selon la revendication 1, 2 ou 3, dans lequel le bromure de tiotropium a un degré de cristallinité inférieur ou égal à 75 %, de préférence le bromure de tiotropium a un degré de cristallinité inférieur ou égal à 70 %.

**5.** Procédé pour la préparation d'un complexe bromure de tiotropium-PVP, le procédé comprenant la préparation d'une solution de bromure de tiotropium, de PVP et d'un solvant, la concentration de la solution sous vide pour obtenir un résidu et le séchage du résidu pour obtenir le complexe bromure de tiotropium-PVP.

**6.** Procédé selon la revendication 5, dans lequel le solvant est choisi dans le groupe constitué par l'acétonitrile, le méthanol, l'eau, le diméthylformamide, l'acétone, le tétrahydrofurane et le diméthylsulfoxyde.

**7.** Procédé selon la revendication 5 ou 6, dans lequel la quantité de PVP va d'environ 0,2 % à environ 90 % en poids du bromure de tiotropium, de préférence la quantité de PVP va d'environ 40 % à environ 60 % en poids du bromure de tiotropium.

**8.** Procédé pour la préparation d'un complexe bromure de tiotropium-PVP, le procédé comprenant la préparation d'une solution de bromure de tiotropium, de PVP et d'un solvant et l'évaporation éclair du solvant.

**9.** Procédé selon la revendication 8, dans lequel le solvant est évaporé par évaporation éclair par application de chaleur et de vide.

**10.** Procédé selon la revendication 8, dans lequel l'évaporation éclair est effectuée par séchage par pulvérisation.

**11.** Procédé pour la préparation d'un complexe bromure de tiotropium-PVP, le procédé comprenant la préparation d'une solution de bromure de tiotropium, de PVP et d'un solvant, la cryodessiccation de la solution et la lyophilisation de la solution cryodesséchée.

**12.** Composition pharmaceutique comprenant un complexe bromure de tiotropium-PVP selon l'une quelconque des revendications 1 à 4, conjointement avec un ou plusieurs excipients pharmaceutiquement acceptables.

**13.** Composition pharmaceutique selon la revendication 12, dans laquelle la formulation est appropriée pour l'administration par inhalation.

**14.** Composition pharmaceutique selon la revendication 12 ou 13, dans laquelle la formulation est un aérosol.

**15.** Composition pharmaceutique selon la revendication 12, 13 ou 14, dans laquelle la formulation comprend un propulseur.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0418716 A
- EP 1326862 A
- EP 1401445 A
- WO 2007075858 A
- WO 2008152398 A